# FASCICULE DE BREVET EUROPEEN

(11) **EP 1 436 026 B1**
(45) Date de publication et mention de la délivrance du brevet: **15.12.2010**
(21) Numéro de dépôt: 02790515.7
(22) Date de dépôt: 15.10.2002
(51) Int. Cl.: A61M 5/32

(54) **DISPOSITIF DE SECURITE POUR UNE SERINGUE**
SICHERHEITSVORRICHTUNG FÜR EINE SPRITZE
SAFETY DEVICE FOR A SYRINGE

(30) Priorité: 15.10.2001 FR 0113256
(43) Date de publication de la demande: 14.07.2004
(73) Titulaire: Rexam Healthcare La Verpillière, 38290 La Verpillière (FR)
(72) Inventeur: RIMLINGER, Thierry, F-38080 L'Isle D'Abeau (FR); POUGET, Michel, F-38300 Domarin (FR); DODIER, Philippe, F-69110 Sainte-Foy-les-Lyon (FR)
(74) Mandataire: de la Bigne, Guillaume Michel Marie
(86) Numéro de dépôt international: PCT/FR2002/003529
(87) Numéro de publication internationale: WO 2003/033059

(56) Documents cités:
- WO-A-00/33900
- WO-A-01/41841
- WO-A-01/60435
- WO-A-93/00949
- FR-A- 2 788 986
- US-A- 5 176 656
- US-A- 5 492 536

## Description

La présente invention a pour objet un dispositif de sécurité pour une seringue, notamment pour une seringue pré-remplie, permettant de protéger l'aiguille après l'injection.

On connaît par la demande de brevet européen EP 0 966 983 un système de sécurité pour une seringue pré-remplie, comportant une chemise de protection de l'aiguille, engagée autour de la seringue et située à l'intérieur d'un fourreau, cette chemise étant apte à coulisser par rapport à l'aiguille et au fourreau. En fin d'injection, un ressort provoque l'expulsion de la chemise de protection hors du fourreau de sorte que cette chemise de protection vienne recouvrir l'aiguille.

Ce système présente un certain nombre d'inconvénients.

En effet, pour provoquer l'expulsion de la chemise, l'utilisateur doit exercer une pression relativement forte sur la tête de piston de la seringue afin de provoquer le franchissement d'une butée du fourreau par une butée de la chemise. Autrement dit, l'utilisateur doit accomplir un geste supplémentaire à la suite du geste produisant l'injection.

De plus, dans le cas où l'utilisateur provoque l'expulsion de la chemise alors que la seringue se trouve encore à proximité du patient, la chemise vient percuter la peau de ce dernier et peut le blesser.

Dans le cas où l'utilisateur éloigne d'abord la seringue avant de provoquer l'expulsion de la chemise, l'aiguille demeure non-protégée pendant le mouvement d'éloignement, ce qui présente un risque de contamination et/ou de blessure accidentelles.

On connaît encore par la demande de brevet français FR 2 799 375 un dispositif d'injection comportant une seringue et un dispositif de protection de l'aiguille après usage. Ce dispositif de protection comporte une bague de verrouillage dans laquelle est fixée la seringue, l'ensemble ainsi formé étant engagé de manière coulissante dans un étui de protection. En fin d'injection, un ressort provoque le déplacement de l'ensemble précité de manière à ce que l'aiguille soit en retrait dans l'étui de protection.

Ce dispositif présente un encombrement axial relativement important.

On connaît encore par la demande internationale WO 00/33900 de la société déposante un ensemble de sécurité pour une seringue pré-remplie, comportant un fourreau tubulaire dans lequel le corps de la seringue, directement inséré, est déplaçable axialement entre une position active d'injection et une position de protection de l'aiguille.

La présente invention vise à proposer un dispositif de sécurité pour une seringue pouvant être actionné, en fin d'injection, de manière simple, sans geste supplémentaire.

La présente invention vise également à proposer un dispositif de sécurité qui soit adapté à au moins deux types de seringues, qui permette un emploi à la fois commode et sécurisant des seringues, et qui soit en outre de réalisation commode et économique.

La présente invention peut concerner un dispositif de sécurité pour une seringue, notamment une seringue pré-remplie, la seringue comportant un corps avec à son extrémité proximale une tête, un porte-aiguille situé à l'extrémité distale de ce corps et muni d'une aiguille, un piston mobile dans le corps et une tige de piston apte à pousser le piston, caractérisé par le fait que le dispositif comporte un manchon apte à recevoir le corps de seringue, le manchon comportant à sa partie proximale des moyens de maintien de tête de seringue, aptes à maintenir des seringues d'au moins deux types différents, et par le fait que le dispositif comporte en outre un fourreau de protection dans lequel peut coulisser l'ensemble constitué de la seringue et du manchon entre une première position permettant l'injection et une deuxième position dans laquelle l'aiguille de la seringue est en retrait à l'intérieur du fourreau de protection, et un élément élastique agissant entre ledit fourreau et ledit ensemble permettant de faire passer, en fin d'injection, ledit ensemble de la première position dans la deuxième position par un mouvement relatif de recul de cet ensemble par rapport au fourreau.

Le dispositif de sécurité selon l'invention, destiné à un usage unique, est adapté aux seringues standards du marché qui sont fabriquées en grande quantité à un coût relativement faible.

De plus, le dispositif de sécurité, du fait de ses moyens de maintien de tête de seringue, est adapté à plusieurs types de seringues, permettant ainsi à un utilisateur de ne disposer que d'un seul type de dispositif de sécurité pour équiper plusieurs types de seringues, notamment des seringues de 0,5 ml et des seringues de 1 ml.

L'élément élastique provoque un mouvement de recul de la seringue et du manchon à l'intérieur du fourreau de protection, ce qui est particulièrement avantageux dans la mesure où le fourreau de protection ne risque pas de percuter la peau du patient et de le blesser, contrairement au système décrit dans le brevet européen 0 966 983 précité dans lequel la chemise de protection est expulsée vers l'avant.

Les moyens de maintien précités sont avantageusement réalisés à l'extrémité proximale du manchon, ce qui permet de réduire l'encombrement axial du dispositif.

Les moyens de maintien peuvent comporter au moins deux pattes de maintien élastiquement déformables reliées à une collerette du manchon, la tête de seringue étant maintenue contre cette collerette par les deux pattes.

En variante, les moyens de maintien comportent une collerette reliée par une pluralité de ponts de matière à une bague supérieure, la tête de seringue étant retenue entre la collerette et la bague supérieure.

La bague supérieure peut définir une ouverture de section axiale généralement tronconique divergeant vers le haut, permettant ainsi de faciliter l'engagement de la tête de seringue dans cette bague.

Le fourreau de protection peut comporter, notamment à son extrémité proximale, des pattes de retenue élastiquement déformables aptes à retenir l'ensemble constitué de la seringue et du manchon dans la première position et à être déformées de manière à libérer ledit ensemble, lui permettant de coulisser axialement par rapport au fourreau et de passer dans la deuxième position.

Les pattes de retenue pouvant être écartées d'une manière relativement simple, sans nécessiter de la part de l'utilisateur qu'il exerce un effort important, celui-ci n'a pas à accomplir de geste supplémentaire autre que celui d'une injection classique pour actionner le dispositif de sécurité.

Les pattes de retenue peuvent servir de butée au manchon de sorte que la seringue solidaire du manchon soit immobilisée en translation par rapport au fourreau de protection lorsque l'utilisateur fait pénétrer l'aiguille sous la peau du patient.

La tige de piston de la seringue peut comporter un poussoir agencé de manière à agir directement sur les pattes de retenue.

On peut ainsi réduire le nombre de pièces constitutives du dispositif de sécurité, ce qui permet d'avoir un coût de revient relativement faible.

En variante, le dispositif comporte un élément de déverrouillage, distinct de la seringue, mobile axialement par rapport au fourreau et apte à agir sur les pattes de retenue.

Les pattes de maintien précitées peuvent comporter chacune une surface sur laquelle vient en appui une patte de retenue.

En variante, les pattes de retenue viennent en appui sur la collerette du manchon.

Les pattes de retenue sont de préférence agencées de manière à être écartées lorsqu'elles sont actionnées.

L'élément de déverrouillage précité peut comporter un bouton, distinct du manchon, rapporté sur le fourreau.

En variante, le bouton est réalisé d'un seul tenant avec le manchon, le bouton pouvant alors comporter une paroi se raccordant au reste du manchon par l'intermédiaire d'une pluralité de bras élastiques.

Le fourreau comporte avantageusement une collerette servant de surface d'appui à des doigts d'un utilisateur.

Le fourreau peut comporter une paroi intérieure pouvant être continue ou, de préférence, discontinue contre laquelle vient en appui l'élément élastique.

Le manchon peut comporter, dans sa partie distale, des ergots aptes à venir en butée contre la portée précitée, lorsque le dispositif passe dans la deuxième position de protection, en fin d'injection.

Dans un exemple de mise en oeuvre de l'invention, le manchon comporte un tube cylindrique à paroi pleine.

Ce tube peut être rigide et comporter, de préférence à son extrémité distale, une pluralité, de préférence deux, ergots précités.

Le fourreau de protection peut comporter une pluralité, de préférence quatre, nervures de guidage longitudinales. Celles-ci sont disposées par paires et les nervures de chaque paire sont espacées d'une distance prédéterminée permettant à un ergot du manchon de s'engager entre elles.

Selon un aspect de l'invention, les nervures sont continues et l'une des nervures de chaque paire peut s'interrompre avant l'autre nervure de manière à définir un passage inférieur permettant d'introduire un ergot du manchon entre les deux nervures par un mouvement de rotation du manchon par rapport au fourreau. L'une des nervures peut par exemple être plus courte que l'autre.

Selon un autre aspect de l'invention, l'une des nervures de chaque paire est discontinue et comporte un passage permettant d'y engager un ergot du manchon et de le positionner entre les deux nervures de la paire et le fourreau peut comporter au moins un relief réalisé sur sa paroi intérieure sur lequel vient buter un ergot au cours du mouvement d'introduction du manchon dans le fourreau et servant à positionner l'ergot en regard du passage précité.

L'introduction de l'ergot dans le passage est obtenue par une rotation du manchon par rapport au fourreau d'un angle prédéterminé, par exemple environ d'un quart de tour lorsque le fourreau comporte deux paires de nervures.

Après cette rotation, lé manchon étant guidé par les nervures de guidage subit un déplacement axial dans le fourreau jusqu'à ce que les pattes de retenue soient engagées sur les moyens de maintien du manchon.

Dans un autre exemple de mise en oeuvre de l'invention, le manchon comporte dans sa partie distale des fentes longitudinales définissant des pattes élastiquement déformables sur lesquelles sont réalisés les ergots précités.

Le dispositif comporte avantageusement des moyens anti-retour s'opposant, lorsque le manchon et la seringue sont dans la deuxième position, à un coulissement de cet ensemble hors du fourreau, ces moyens pouvant comporter des pattes de blocage élastiquement déformables réalisées sur le fourreau de protection, ces pattes de blocage étant aptes à être franchies par les ergots précités du manchon lorsque celui-ci passe de la première dans la deuxième position et servant de butée à ces ergots après le franchissement.

Le fourreau, et respectivement le manchon, peuvent être constitués d'une pièce réalisée d'un seul tenant.

L'élément élastique, un ressort hélicoïdal par exemple, peut être en appui sur une surface du manchon.

Le fourreau de protection peut être réalisé dans une matière transparente ou semi-transparente.

L'invention a pour objet un dispositif de sécurité pour une seringue à aiguille, du type décrit dans US-A-5 176 656 ou US-A-5 492 536, qui comporte un fourreau de protection dans lequel peut coulisser la seringue, ledit fourreau présentant à une partie distale radialement déformable élastiquement une ouverture apte à permettre à l'aiguille de la seringue de dépasser du fourreau, **caractérisé en ce que** le fourreau est, à sa partie distale, radialement déformable élastiquement, entre une configuration de repos où son ouverture présente un diamètre minimum, et une configuration déformée permettant le passage, au travers de l'ouverture, d'un corps présentant un diamètre supérieur au diamètre minimum de l'ouverture.

Ce corps est par exemple un capuchon destiné à protéger l'aiguille de la seringue. Dans ces conditions, le fourreau permet d'assurer un maintien ferme du capuchon avant l'utilisation de la seringue, ce qui évite tout accident et met l'aiguille à l'abri des contaminations préalables à l'injection.

Il peut également s'agir du corps de la seringue, en particulier lorsque celle-ci est sur le point d'être utilisée. Il en résulte un maintien ferme de la seringue dans le fourreau, au bénéfice de la sécurité et de la précision de l'injection.

Le fourreau pouvant être réalisé par moulage, le corps dont il est question ci-dessus peut également être une partie mobile d'un moule employé pour la réalisation du fourreau. L'élasticité radiale du fourreau permet le retrait aisé de la partie mobile du moule lors du démoulage.

Suivant un mode de réalisation, le fourreau présente, à sa partie distale, une forme tronconique.

Par ailleurs, la partie distale du fourreau peut être fendue, afin de favoriser l'élasticité radiale du fourreau. Ainsi, suivant un mode de réalisation, la partie distale du fourreau comporte plusieurs fentes longitudinales définissant des languettes élastiques convergentes.

Le dispositif de sécurité peut en outre comporter des moyens pour permettre à la seringue de coulisser dans le fourreau entre une première position permettant l'injection et une deuxième position dans laquelle l'aiguille de la seringue est en retrait à l'intérieur du fourreau.

Suivant un mode de réalisation, le dispositif de sécurité comporte un manchon apte à recevoir le corps de la seringue, le manchon comportant à sa partie proximale des moyens de maintien de la tête de la seringue aptes à maintenir des seringues d'au moins deux types différents, l'ensemble constitué de la seringue et du manchon pouvant coulisser entre la première et la deuxième position.

Les moyens de maintien sont de préférence agencés pour assurer le maintien d'une collerette formant la tête de la seringue.

Il peut en outre être prévu un élément élastique agissant entre le fourreau et l'ensemble constitué du manchon et de la seringue, et permettant de faire passer, en fin d'injection, ledit ensemble de la première position dans la deuxième par un mouvement relatif de recul de cet ensemble par rapport au fourreau.

L'invention a également pour objet un ensemble comportant une seringue et un dispositif de sécurité tel que décrit ci-dessus, équipant ladite seringue.

Cet ensemble peut également comporter un capuchon protégeant l'aiguille de la seringue. Ce capuchon est par exemple monté de telle manière qu'il dépasse du fourreau au travers de l'ouverture distale de celui-ci.

Le fourreau et le capuchon sont de préférence agencés de manière à empêcher, après retrait du capuchon, la réintroduction de celui-ci par l'ouverture du fourreau.

L'invention sera mieux comprise à la lecture de la description détaillée qui va suivre, d'exemples de mises en oeuvre non limitatifs, et à l'examen du dessin annexé, sur lequel :
- la figure 1 représente schématiquement et partiellement, en coupe axiale, une seringue équipée d'un dispositif de sécurité conforme à un premier exemple de mise en oeuvre de l'invention, avant l'injection,
- la figure 2 représente schématiquement et partiellement, en coupe axiale suivant II-II, la seringue et le dispositif de la figure 1,
- la figure 3 représente schématiquement et partiellement, en perspective, le fourreau de protection du dispositif de la figure 1,
- la figure 4 est une vue schématique et partielle, en perspective et en coupe axiale, du fourreau de la figure 3,
- la figure 5 est une vue analogue à celle de la figure 4 d'un fourreau conforme à une variante de mise en oeuvre de l'invention,
- la figure 6 est une vue schématique et partielle, en perspective, du manchon du dispositif de la figure 1,
- la figure 7 est une vue schématique et partielle, en perspective, de la seringue et du dispositif de la figure 1, avant-l'éur assemblage,
- la figure 8 est une vue analogue à celle de la figure 2, en fin d'injection,
- la figure 9 est une vue analogue à celle de la figure 2, après l'injection, l'aiguille étant en retrait dans le fourreau de protection,
- la figure 10 est une vue analogue à celle de la figure 2, la seringue ayant des dimensions différentes de la seringue de la figure 2,
- la figure 11 représente schématiquement et partiellement, en coupe axiale, une seringue équipée d'un dispositif de sécurité conforme à un autre exemple de mise en oeuvre de l'invention, avant l'injection,
- la figure 12 est une vue analogue à celle de la figure 11, après l'injection, l'aiguille étant en retrait dans le fourreau de protection,
- la figure 13 représente schématiquement et partiellement, en perspective, le manchon du dispositif de sécurité de la figure 11,
- la figure 14 représente schématiquement et partiellement, en coupe axiale, le manchon de la figure 13,
- la figure 15 représente schématiquement et partiellement, en coupe axiale, une seringue équipée d'un dispositif de sécurité conforme à un autre exemple de mise en oeuvre de l'invention,
- la figure 16 représente schématiquement et partiellement, en coupe axiale, une seringue équipée d'un dispositif de sécurité conforme à un autre exemple de mise en oeuvre de l'invention,
- la figure 17 représente schématiquement et partiellement, en coupe axiale, le manchon d'un dispositif tel que représenté sur les figures précédentes, conformément à un autre exemple de mise en oeuvre,
- la figure 18 est une vue analogue à la figure 17, suivant une variante d'exécution, et
- la figure 19 représente schématiquement et partiellement, en perspective et en coupe axiale, le manchon de la figure 18.

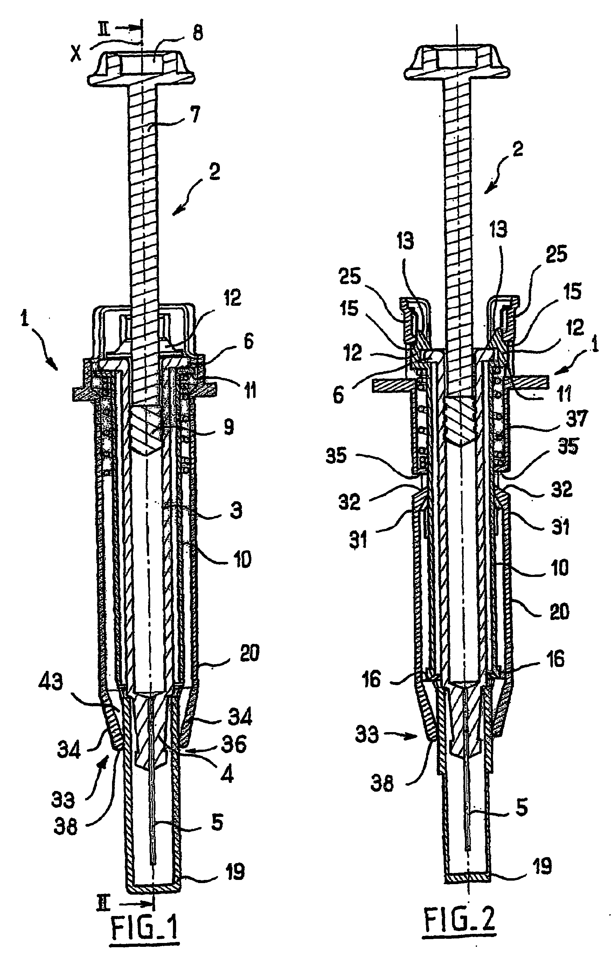

On a représenté sur la figure 1 un dispositif de sécurité 1 à usage unique conforme à l'invention, équipant une seringue pré-remplie 2.

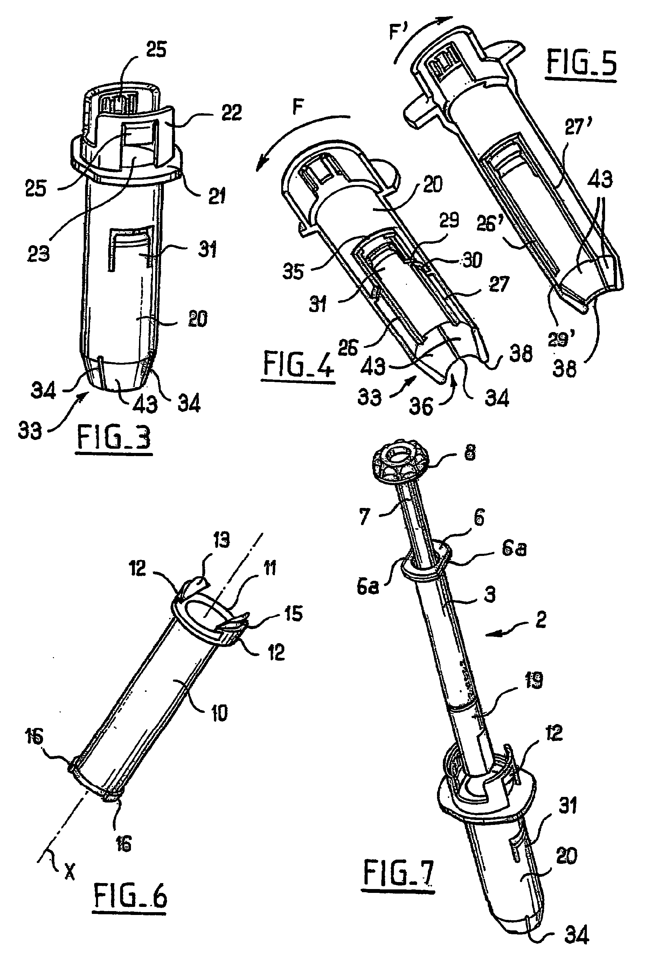

La seringue 2 présente une contenance de 0,5 ml. Celle-ci comporte un corps 3 d'axe X et muni à son extrémité distale d'un porte-aiguille 4 sur lequel est montée une aiguille 5. Le corps de seringue 3 comporte à son extrémité proximale une tête ou collerette 6. Cette dernière présente deux bords droits parallèles 6a, comme on peut le voir sur la figure 7. Un piston 9 mobile à l'intérieur du corps de seringue sert à injecter,le produit contenu dans celui-ci, ce piston 9 étant actionné par une tige de piston 7 terminée par un poussoir 8 sur lequel l'utilisateur exerce une poussée pour injecter le produit.

Dans la suite de la description, les termes « haut » et « bas » se réfèrent à l'orientation choisie sur les figures 1 et 2, où l'aiguille 5 de la seringue est tournée vers le bas de la page.

L'aiguille 5 est initialement protégée par un capuchon 19 que l'utilisateur retire au moment de l'injection.

Le dispositif de sécurité 1 comporte un manchon 10 dans lequel est engagé le corps de seringue 3.

Le manchon 10, représenté isolément sur la figure 6, est constitué, sur une majeure partie de sa longueur, d'un tube cylindrique de révolution d'axe X, à paroi pleine. Le manchon 10 comporte à son extrémité proximale une collerette 11 et deux pattes de maintien élastiquement déformables 12, diamétralement opposées et se raccordant à la collerette 11 sensiblement à la périphérie de celle-ci.

Ces pattes 12 permettent de maintenir la tête de seringue 6 sur la collerette 11.

1 Chaque patte 12 comporte, à sa partie supérieure, un flanc incliné 13 permettant à la tête de seringue 6 de franchir facilement les pattes 12 et de s'y encliqueter.

A l'arrière de chacun des flancs 13, est réalisée une encoche 15 dont le rôle est expliqué plus loin.

Le manchon 10 comporte, à son extrémité distale et sur sa paroi extérieure deux ergots 16 diamétralement opposés.

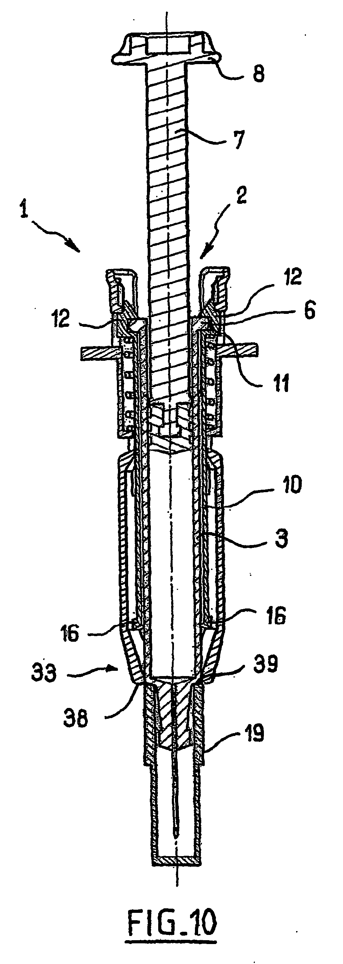

Le diamètre du manchon 10 et les dimensions des pattes 12 sont choisis de sorte que le manchon puisse recevoir deux types de seringues, par exemple des seringues de type 0,5 ml et des seringues de type 1 ml, le dispositif 1 équipé d'une seringue de 1 ml étant représenté sur la figure 10.

Le manchon 10 est destiné à être introduit dans un fourreau de protection 20 représenté isolément sur la figure 3. Ce fourreau de protection 20 comporte, à sa partie proximale, une collerette 21 sur laquelle se raccorde une paroi supérieure généralement tubulaire 22. La collerette 21 sert de surface d'appui pour des doigts d'un utilisateur procédant à l'injection.

La paroi tubulaire 22 comporte deux fenêtres 23 présentant chacune un bord supérieur auquel se raccorde une patte de retenue 25. Cette dernière est apte à être écartée radialement par déformation élastique.

La partie inférieure des fenêtres 23, non occupée par les pattes de retenue 25, ménage un passage pour les pattes de maintien 12 du manchon 10.

Comme on peut le voir sur la figure 2, lorsque le manchon 10 est en place dans le , fourreau 20, les pattes de retenue 25 viennent prendre appui chacune sur une encoche 15 des pattes de maintien 12, permettant ainsi de retenir le manchon 10 dans le fourreau 20.

Le poussoir 8 présente un diamètre suffisant de manière à venir au contact des pattes de retenue 25 et les écarter, en fin d'injection.

Le fourreau de protection 20 comporte, réalisées sur sa paroi intérieure, quatre nervures de guidage longitudinales disposées par paire, les nervures 26 et 27 d'une même paire ménageant entres elles un espace permettant aux ergots 16 du manchon 10 de s'y engager.

La nervure 27 est interrompue de manière à former un passage 29. A droite de cette nervure 27 est réalisée une butée 30 positionnée par rapport au passage 29 de sorte que lorsqu'un ergot vient en appui sur cette butée 30, celui-ci se retrouve en regard du passage 29 et peut s'y engager à la suite d'une rotation du manchon 10 par rapport au fourreau 20.

Le fourreau 20 comporte également deux pattes de blocage élastiquement déformables 31 réalisées dans sa paroi.

Les pattes de blocage 31, situées chacune entre deux nervures 26 et 27 d'une paire, comportent chacune une extrémité recourbée 32 dirigée vers l'intérieur.

Les extrémités recourbées 32 sont situées au-dessus des butées 30 de sorte que, lors du montage du manchon 10 dans le fourreau 20, les ergots 16 n'aient pas à franchir les pattes de blocage 31.

Le fourreau de protection 20 présente, à sa partie distale 33, une forme généralement tronconique convergeant vers le bas. Cette partie comporte quatre fentes 34 lui conférant une certaine élasticité radiale et lui permettant ainsi de recevoir le capuchon 19 ou, comme nous le verrons ci-après, un autre corps, de diamètre supérieur à celui de l'ouverture distale 36 du fourreau 20.

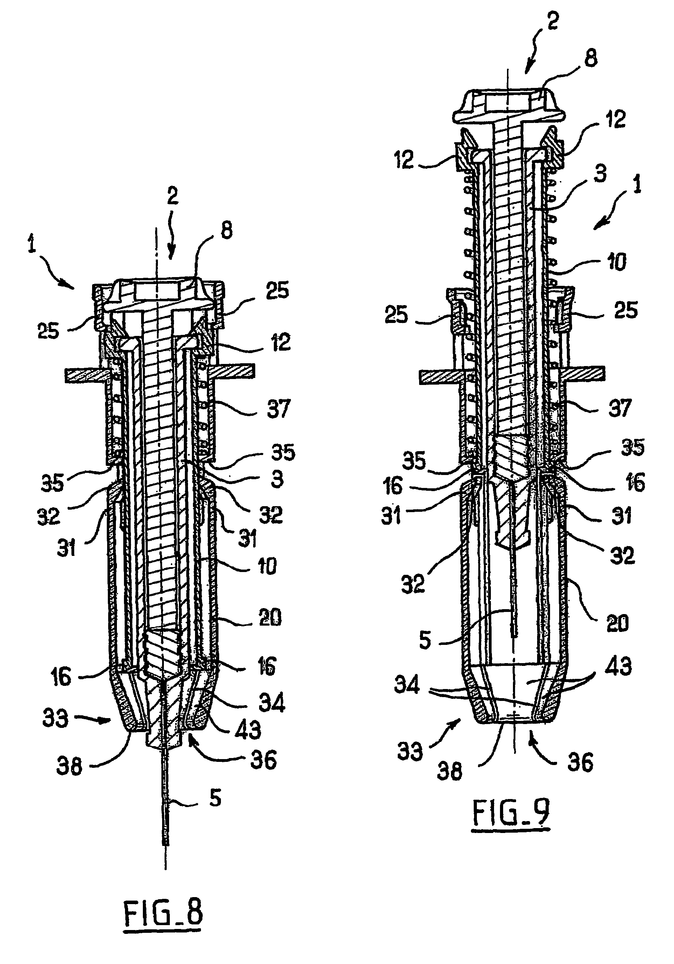

Plus précisément, le fourreau 20 est, à sa partie distale, radialement déformable, entre une configuration de repos, illustrée sur la figure 9, où son ouverture 36 présente un diamètre minimum, et une configuration déformée, illustrée notamment sur la figure 1, permettant le passage, au travers de l'ouverture 36, d'un corps présentant un diamètre supérieur au diamètre minimum de l'ouverture 36.

Comme nous venons de le voir, ce corps peut être le capuchon 19 venant protéger l'aiguille 5 de la seringue 2, comme cela est représenté sur la figure 1 où l'on voit que, avant l'injection, le capuchon 19 est monté de telle manière qu'il dépasse du fourreau 20 au travers de l'ouverture 36.

Le fourreau 20 enserre ainsi le capuchon 19, et exerce sur lui une force qui s'oppose à son retrait, suffisante pour garantir que le capuchon 19 ne sera pas séparé intempestivement de la seringue 2.

II en résulte une diminution des risques de blessure et de contamination dues à un contact accidentel de l'aiguille 5 avec une personne autre que le patient auquel est destinée l'injection.

De plus, comme cela est visible sur la figure 10, le fourreau 20 et le capuchon 19 sont agencés pour empêcher, après retrait du capuchon 19, la réintroduction de celui-ci dans le fourreau 20 par son ouverture 36.

En effet, une fois retiré le capuchon 19, la partie distale du fourreau 20 se resserre de telle manière que l'ouverture 36 présente un diamètre inférieur au diamètre extérieur du capuchon 19. En l'occurrence, la partie distale du fourreau vient enserrer le corps de seringue 3.

Plus précisément, bien que l'ouverture 36 présente un bord arrondi 38, le capuchon 19 présente, du côté de son ouverture, un bord droit 39 qui vient buter contre le bord arrondi 38 de l'ouverture 36.

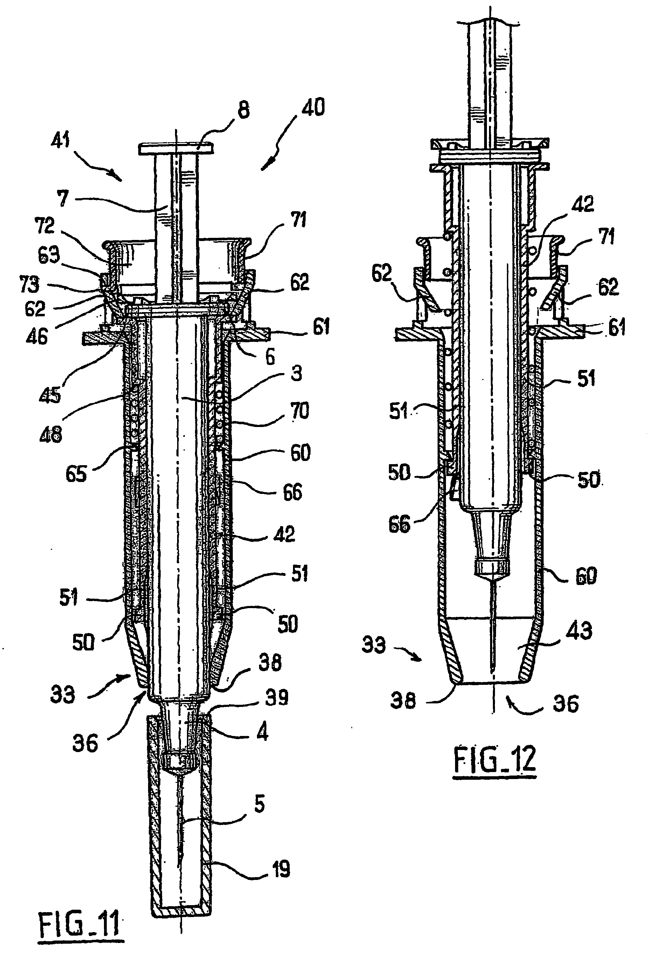

Un autre corps pouvant être reçu dans le fourreau 20 au travers de l'ouverture peut être constitué par le corps de seringue 3, comme cela est représenté sur les figures 10 et 11 où, comme nous le verrons ci-après, est représentée une seringue 2 de taille supérieure (en diamètre et en longueur) à la seringue 2 représentée sur les figures 1 et 2.

Comme cela est visible sur les figures 11 et 12, le corps de seringue 3 présente une longueur telle que, dans la configuration précédant l'injection et pendant l'injection, il dépasse du fourreau 20 au travers de l'ouverture 36.

Grâce à son élasticité radiale à sa partie distale 33, le fourreau 20 enserre le corps de seringue 3 qui présente ici un diamètre supérieur au diamètre minimum de l'ouverture 36. Il en résulte un maintien ferme du corps de seringue 3 à proximité du porte aiguille 4.

Ce maintien, combiné au maintien de la tête de seringue 6 assuré par le manchon 10, garantit une tenue rigide de la seringue 2 au sein du fourreau 20, au bénéfice de la sécurité et de la précision des opérations d'injection.

Le fourreau 20 peut être fabriqué par moulage. Le moule employé (non représenté) comporte par exemple une partie extérieure fixe, creuse, au sein de laquelle coulisse une partie mobile sensiblement cylindrique, l'empreinte du moule étant formée entre la partie fixe et la partie mobile.

Ainsi, un autre corps pouvant être reçu dans le fourreau 20 au travers de l'ouverture 36 peut être constitué par la partie mobile du moule, qui, lors du démoulage, est retirée du fourreau 20 selon la direction axiale, du côté de l'ouverture 36. Ce retrait est facilité par l'élasticité radiale de la partie distale 33 du fourreau 20.

Comme nous l'avons vu, afin de permettre cette élasticité radiale, la partie distale 33 du fourreau 20 est percée d'une pluralité de fentes 34, ici au nombre de quatre, bien qu'un nombre différent (au moins deux, et par exemple six) puisse être envisagé.

Ces fentes 34, qui s'étendent longitudinalement et débouchent sur l'ouverture 36, définissent des languettes 43 élastiquement flexibles qui convergent vers le bas et confèrent au fourreau 20 son élasticité radiale.

Le fourreau 20 comporte en outre deux épaulements transversaux intérieurs 35 s'étendant entre les nervures de guidage 26 et 27 d'une paire et situés au-dessus des extrémités recourbées 32 dés pattes de blocage 31.

Le dispositif 1 comporte un ressort hélicoïdal 37 mis en place autour du manchon 10 et en appui à une extrémité, contre les épaulements 35 et, à l'autre extrémité, contre la collerette 11.

Le montage du manchon 10 dans le fourreau 20 s'effectue de la manière suivante.

D'abord, on oriente le manchon 10 par rapport au fourreau 20 de manière à ce que les deux pattes de maintien 12 soient positionnées entre les deux pattes de retenue 25 du fourreau 20.

On introduit le manchon 10 dans le fourreau 20 tout en respectant cette orientation, ce premier mouvement d'introduction se poursuivant jusqu'à ce que les ergots 16 du manchon 10 viennent en appui contre les butées 30 du fourreau 20.

On procède alors à une rotation d'un quart de tour dans le sens de la flèche F, représentée sur la figure 4, du manchon 10 par rapport au fourreau 20 de manière à faire passer chaque ergot 16 à travers un passage 29.

A l'issue de cette rotation, l'ergot 16 vient se loger entre les nervures 26 et 27 et un second mouvement d'introduction du manchon 10 dans le fourreau 20 est effectué. Les ergots 16 engagés entre les nervures 26 et 27 assurent alors le guidage du manchon 10 par rapport au fourreau 20. Ce mouvement se poursuit jusqu'à ce que les pattes de maintien 12 viennent s'encliqueter sous les pattes de retenue 25.

Une fois le manchon 10 et le fourreau 20 assemblés, la seringue pré-remplie 2 est introduite dans le manchon 10, en orientant la tête de seringue 6 de sorte que les côtés droits 6a viennent en regard des pattes de maintien 12, comme on peut le voir sur la figure 7.

La tête 6 vient s'encliqueter sur les pattes de maintien 12 de manière à être retenue sur la collerette 11.

L'engagement des ergots 16 entre les nervures 26 et 27 permet un blocage en rotation du manchon 10 par rapport au fourreau 20.

Dans l'exemple qui vient d'être décrit, l'une des nervures est interrompue. En variante, comme illustré à la figure 5, toutes les nervures peuvent être continues et l'une des nervures 26' peut s'interrompre avant l'autre nervure 27', la nervure 26' étant plus courte que l'autre.

Un passage inférieur 29' est ainsi ménagé en dessous de la nervure 26', permettant d'introduire un ergot 16 du manchon 10 entre les deux nervures 26' et 27' par un mouvement de rotation d'un quart de tour, suivant la flèche F', du manchon 10 par rapport au fourreau 10.

Dans cet exemple de mise en oeuvre, sont nécessaires un seul mouvement axial d'introduction suivi d'une rotation.

Le fonctionnement du dispositif de sécurité 1 s'effectue de la manière suivante.

Pour injecter le produit contenu dans la seringue 2, l'utilisateur exerce une poussée sur le poussoir 8 de manière à enfoncer le piston 9.

En fin de course du piston 9 dans le corps de seringue 3, le poussoir 8 provoque l'écartement des pattes de retenue 25 de manière à libérer le manchon 10 et le ressort 37 provoque le recul de l'ensemble constitué du manchon 10 et de la seringue 2 vers l'arrière, comme on peut le voir sur la figure 8.

Lors de ce mouvement de recul, les ergots 16 du manchon 10 franchissent les pattes de blocage 31 du fourreau 20.

En fin de course, comme illustré sur la figure 9, les ergots 16 viennent en butée contre les épaulements 35 et l'aiguille 5 est en retrait dans le fourreau 20.

Les ergots 16 qui sont engagés au-dessus des extrémités recourbées 32 des pattes 31 empêchent un déplacement vers l'extrémité distale du manchon 10 par rapport au fourreau 20.

On assure ainsi le verrouillage du dispositif 1 dans sa position de sécurité, après injection.

On a représenté en référence aux figures 11 à 14 un dispositif de sécurité 40 conforme à un autre exemple de mise en oeuvre de l'invention.

Ce dispositif de sécurité 40 est agencé de manière à recevoir une seringue standard 41. de contenance de 0,5 ml ou 1 ml, sensiblement analogue à la seringue 2 précédemment décrite.

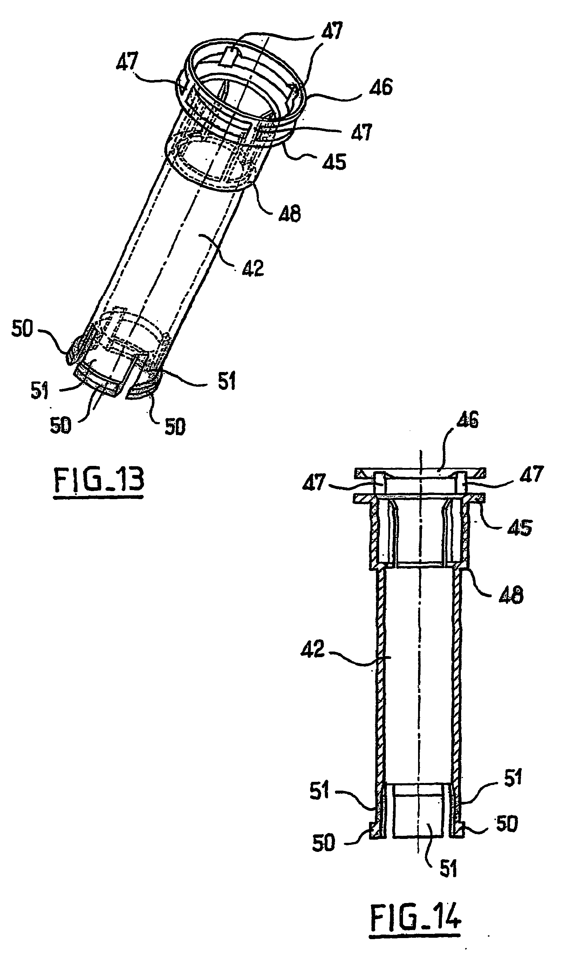

Le dispositif de sécurité 40 comporte un manchon 42 dans lequel est engagé le corps de seringue 3. Comme on peut le voir notamment sur les figures 13 et 14, le manchon 42 comporte à son extrémité proximale des moyens de maintien de la collerette ou tête 6 du corps de seringue. Ces moyens de maintien comprennent une collerette 45 reliée par une pluralité de ponts de matière 47, par exemple quatre ponts de matière 47, à une bague supérieure 46, la tête de seringue 6 étant retenue entre la collerette 45 et cette bague 46.

La bague 46 définit une ouverture supérieure présentant en coupe axiale une forme généralement tronconique divergeant vers le haut permettant de guider la tête de seringue 6 à travers cette ouverture lors du montage de la seringue dans le manchon 42.

Le manchon 42 comporte en outre un épaulement 48 définissant un élargissement de section dans sa partie proximale.

Le manchon 42 comporte à son extrémité distale une pluralité de pattes élastiquement déformables 51 d'épaisseur réduite, à l'extrémité de chacune desquelles est réalisé un ergot 50.

Après leur assemblage, la seringue 2 et le manchon 42 sont immobilisés l'un par rapport à l'autre.

Le manchon 42 est destiné à être engagé dans un fourreau de protection 60 comportant dans sa partie proximale une collerette 61 dont la surface inférieure sert de surface d'appui à des doigts d'un utilisateur lors de l'injection.

Le fourreau 60 comporte à son extrémité proximale, au-dessus de la collerette 61, une pluralité de pattes de retenue élastiquement déformables 62 repliées, au repos, vers l'intérieur.

Entre les pattes 62 sont ménagées des ouvertures 63 dont le rôle est expliqué plus loin.

Les pattes de retenue 62, à l'état replié, viennent en appui contre la collerette 45 du manchon 42, limitant ainsi le déplacement axial vers le haut de l'ensemble constitué du manchon 42 et de la seringue 2.

Le fourreau de protection 60 comporte également, sensiblement à mi-longueur dans l'exemple décrit, une portée intérieure discontinue 65. La tranche de la portée 65 est généralement tronconique divergeant vers le haut, permettant, lors de l'introduction du manchon 10 dans le fourreau 60, de guider les ergots 50 à travers cette portée 65.

Le dispositif 40 comporte des moyens anti-retour ou de verrouillage définis par des pattes de blocage 66, sensiblement analogues aux pattes de blocage 31 précédemment décrites et réalisées dans la paroi du fourreau 60.

Ces pattes de blocage 66 peuvent être franchies par les ergots 50.

Un ressort hélicoïdal 70 est disposé dans le fourreau 60, autour du manchon 42, en appui respectivement sur la portée 65 et l'épaulement 48.

Le dispositif de sécurité 40 comprend également un bouton 71 comportant une paroi cylindrique 72 avec à son extrémité distale des crochets 73 aptes à s'engager dans les ouvertures 63 précitées du fourreau 60, situées entre les pattes 62.

La paroi cylindrique 72 présente à son extrémité proximale un rebord divergeant vers le haut, sur lequel un utilisateur vient appliquer son pouce pour enfoncer le poussoir 71.

Le bord distal du bouton 71 est destiné à venir au contact des pattes 62 et les écarter.

Le fonctionnement du dispositif de sécurité 40 s'effectue de la manière suivante.

L'utilisateur introduit l'aiguille 5 sous la peau du patient, la seringue étant immobilisée en translation par rapport au fourreau 60. Pour injecter le contenu de la seringue, l'utilisateur exerce, au moyen de l'index et du majeur qui viennent en appui sur la collerette 61 et au moyen de son pouce en appui sur la tête 8, une poussée suffisante pour injecter le produit.

En fin de course de la tige de piston 7 dans le corps 3, l'utilisateur, au moyen de son pouce qui est en appui sur la tête de piston 8, vient enfoncer le bouton 71 sans effectuer de geste supplémentaire autre que celui produisant l'injection, de manière à écarter les pattes 62 et libérer le manchon 42. L'ensemble constitué de la seringue 2 et du manchon 42 peut alors être entraîné vers le haut sous l'action du ressort 70.

Lors de ce déplacement vers le haut, les ergots 50 franchissent les pattes de blocage 66 et viennent en butée contre la portée 65, limitant ainsi la course de l'ensemble constitué de la seringue 2 et du manchon 42 vers le haut, comme on peut le voir sur la figure 12.

L'aiguille 5 est alors en retrait à l'intérieur du fourreau de protection 60, ne présentant ainsi plus aucun danger de contamination et/ou de blessure après l'injection.

Par ailleurs, les ergots 50 engagés au-dessus des pattes 66 empêchent la seringue d'être repoussée hors du fourreau de protection 60, permettant ainsi un verrouillage du dispositif de sécurité 1 après usage.

Dans l'exemple décrit, le bouton 71 est constitué par un élément rapporté fixé sur le fourreau de protection 60.

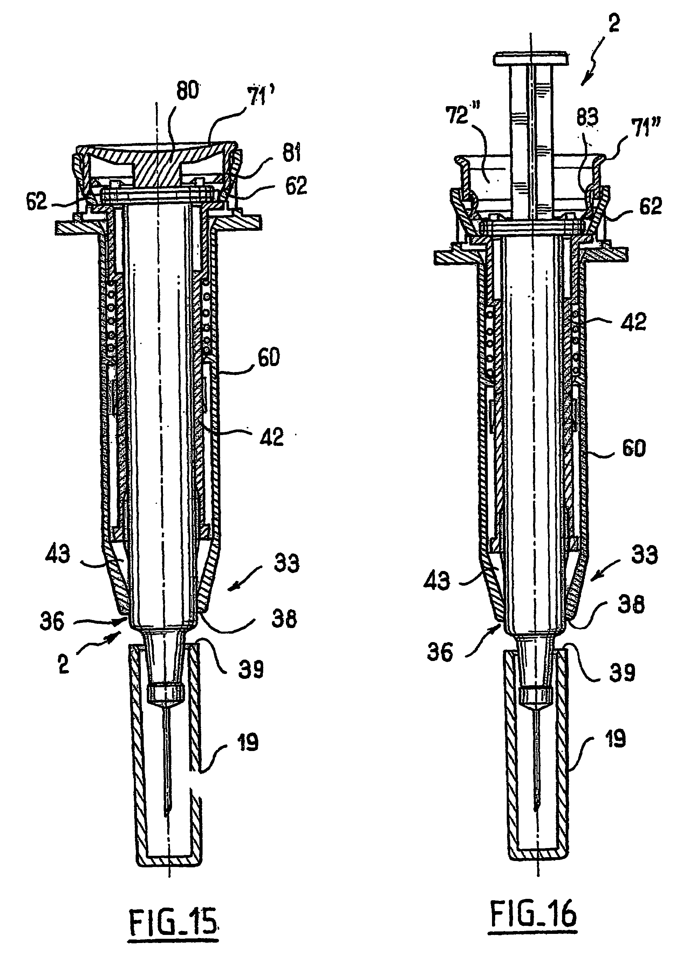

En variante, comme cela est illustré à la figure 15, le dispositif de sécurité comporte un bouton 71' réalisé d'un seul tenant avec la tête 8 de la tige de piston 7. Ce poussoir 71' comporte à sa partie supérieure une surface 80 concave permettant à un utilisateur d'y appliquer son pouce.

Le bouton 71' comporte une jupe 81 s'étendant sous la surface 80 et permettant, lorsque la tige de piston 7 est sensiblement en fin de course d'injection, d'écarter les pattes de retenue 62 et de libérer l'ensemble constitué de la seringue 2 et du manchon 42.

En variante encore, comme illustré à la figure 16, le dispositif de sécurité comporte un bouton 71". Ce dernier comporte une paroi cylindrique 72", laquelle se raccorde au manchon 42 par l'intermédiaire de bras élastiquement déformables 83.

Dans l'exemple décrit, la paroi cylindrique 72", les bras élastiques 83 et le manchon 42 sont réalisés d'un seul tenant.

En fin d'injection, le poussoir 71" est enfoncé et la paroi 72" provoque l'écartement des pattes 62 de manière à libérer le manchon 42.

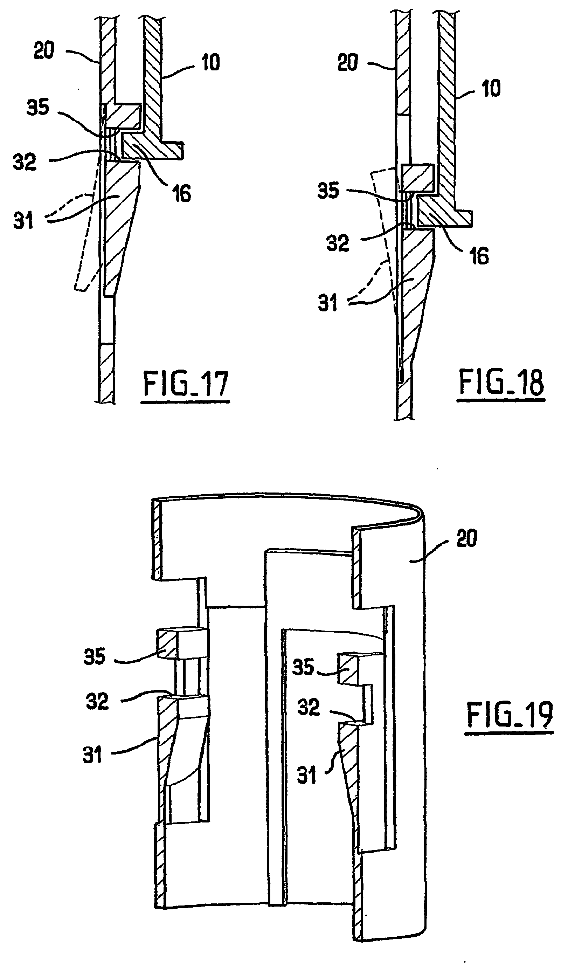

Sur les figures 17 à 19 sont représentées deux variantes d'exécution, conformément auxquelles l'épaulement 35 et l'extrémité recourbée 32 appartiennent tous deux à la même patte élastique 31 propre à être écartée vers l'extérieur, comme cela est représenté sur les figures 17 et 18 en traits interrompus, sous l'effet d'une sollicitation radiale exercée depuis le centre vers l'extérieur du fourreau 20.

Cette particularité permet une introduction plus aisée du manchon 10 lors de l'assemblage du dispositif 1. En effet, en écartant vers l'extérieur les pattes 31, on écarte les épaulements 35 du trajet des ergots 16, qui franchissent sans difficulté les pattes 31 pour venir se positionner dans la partie distale 33 du fourreau 20 (configuration représentée notamment sur la figure 8).

De la sorte, il n'est pas nécessaire de respecter une orientation particulière du manchon 10 lors de son introduction dans le fourreau 20.

A l'inverse, lorsque après l'injection le manchon 10 recule dans le fourreau, les ergots 16 franchissent les pattes de blocage 31 en les écartant vers l'extérieur, jusqu'à venir se positionner, comme cela est représenté sur les figures 17 et 18, dans l'espace ménagé entre les parties 32 et les épaulements 35, le manchon 10 se trouvant alors bloqué dans la position ainsi réalisée, qui opère le verrouillage du dispositif 1 dans sa position de sécurité.

Cette particularité de réalisation permet également une fabrication simplifiée du fourreau 20, car il n'est pas nécessaire de prévoir un moule extérieur en deux parties dont te plan de joint s'étendrait entre les épaulements 35 et les parties 32 des pattes 31.

En effet, compte tenu de l'élasticité radiale des pattes 31 incluant à la fois les parties 32 et les épaulements 35, le moulage du fourreau 20 peut être réalisé au moyen de deux parties de moules, dont l'une, extérieure, fixe, est d'un seul tenant, et dont l'autre, intérieure, mobile à translation dans la partie extérieure, peut être retirée vers la partie proximale du fourreau 20 en écartant sur son passage les pattes 31.

Comme cela est visible sur les figures 17 à 19, les pattes 31 peuvent être rattachées à une partie supérieure du fourreau 20, située du côté de sa partie proximale (figure 17), comme à une partie inférieure du fourreau 20, située du côté de sa partie distale 33 (figures 18 et 19).

## Revendications

1. Dispositif de sécurité (1) pour une seringue (2) à aiguille (5), qui comporte un manchon (10) apte à recevoir le corps (3) de la seringue (2) et un fourreau (20) de protection dans lequel peut coulisser la seringue (2), et des moyens pour permettre à la seringue (2) et au manchon de coulisser dans le fourreau (20) entre une première position permettant l'injection et une deuxième position dans laquelle l'aiguille (5) de la seringue (2) est en retrait à l'intérieur du fourreau (20), ledit fourreau (20) présentant à une partie distale (33) radialement déformable élastiquement une ouverture (36) apte à permettre à l'aiguille (5) de la seringue (2) de dépasser du fourreau (20), **caractérisé en ce que** le fourreau (20) est, à sa partie distale (33), radialement déformable élastiquement, entre une configuration de repos où son ouverture (36) présente un diamètre minimum, et une configuration déformée permettant le passage, au travers de l'ouverture (36), d'un corps (3, 19) présentant un diamètre supérieur au diamètre minimum de l'ouverture (36).

2. Dispositif selon la revendication 1, **caractérisé en ce que** ledit corps (19) est un capuchon destiné à protéger l'aiguille (5) de la seringue (2).

3. Dispositif selon la revendication 1, **caractérisé en ce que** ledit corps (3) est le corps de la seringue (2).

4. Dispositif selon la revendication 1, **caractérisé en ce que**, le fourreau étant réalisé par moulage, ledit corps est une partie mobile d'un moule.

5. Dispositif selon l'une des revendications 1 à 4, **caractérisé en ce que** le fourreau (20) présente, à sa partie distale (33), une forme tronconique.

6. Dispositif selon l'une des revendications 1 à 5, **caractérisé en ce que** la partie distale (33) du fourreau (20) est fendue.

7. Dispositif selon la revendication 6, **caractérisé en ce que** la partie distale (33) du fourreau (20) comporte plusieurs fentes (34) longitudinales définissant des languettes (43) élastiques convergentes.

8. Dispositif selon l'une quelconque des revendications précédentes, **caractérisé en ce que** le manchon (10) comporte à sa partie proximale des moyens de maintien de la tête (6) de la seringue (2) aptes à maintenir des seringues d'au moins deux types différents,

9. Dispositif selon la revendication 8, **caractérisé en ce que** les moyens de maintien sont agencés pour assurer le maintien d'une collerette (6) formant la tête de la seringue (2).

10. Dispositif selon l'une quelconque des revendications précédentes, **caractérisé en ce qu'**il comporte un élément élastique (37) agissant entre le fourreau (20) et l'ensemble constitué du manchon (10) et de la seringue (2), et permettant de faire passer, en fin d'injection, ledit ensemble de la première position dans la deuxième par un mouvement relatif de recul de cet ensemble par rapport au fourreau (20).

11. Ensemble comportant une seringue (2) et un dispositif de sécurité (1) équipant ladite seringue (2), **caractérisé en ce que** ledit dispositif de sécurité (1) est conforme à l'une des revendications 1 à 10.

12. Ensemble selon la revendication 11, **caractérisé en ce qu'**il comporte en outre un capuchon (19) protégeant l'aiguille (5) de la seringue (2).

13. Ensemble selon la revendication 12, **caractérisé en ce que** ledit capuchon (19) est monté de telle manière qu'il dépasse du fourreau (20) au travers de l'ouverture distale (36) de celui-ci.

14. Ensemble selon la revendication 13, **caractérisé en ce que** le fourreau (20) et le capuchon (19) sont agencés de manière à empêcher, après retrait du capuchon (19), la réintroduction de celui-ci par l'ouverture (36) du fourreau (20).

## Claims

1. A safety device (1) for a syringe (2), the device comprising a sleeve (10) suitable for receiving the body (3) of the syringe (2) and a protective sheath (20) in which the syringe (2) can slide, and means for enabling the syringe (2) and the sleeve to slide in the sheath (20) between a first position enabling injection to be performed and a second position in which the needle (5) of the syringe (2) is withdrawn into the inside of the sheath (20), said sheath (20) presenting, at a distal portion (33) that is radially elastically deformable, an opening (36) that is suitable for allowing the syringe (2) to project beyond the sheath (20), the device being **characterized in that** the sheath (20) is, at its distal portion (33), radially elastically deformable, between an not-deformed position, wherein its opening (36) presents a minimal diameter, and a deformed position enabling the passage, through its opening (36), of a body (3, 19) of diameter greater than the minimal diameter of the opening (36).

2. A device according to claim 1, **characterized in that** said cylinder (19) is a cap for protecting the needle (5) of the syringe (2).

3. A device according to claim 1, **characterized in that** said cylinder (3) is the cylinder of the syringe (2).

4. A device according to claim 1, **characterized in that** the sheath is made by molding, and said cylinder is a moving portion of a mold.

5. A device according to any one of claims 1 to 4, **characterized in that** the shape of the distal portion (33) of the sheath (20) is frustoconical.

6. A device according to any one of claims 1 to 5, **characterized in that** the distal portion (33) of the sheath (20) is split.

7. A device according to claim 6, **characterized in that** the distal portion (33) of the sheath (20) has a plurality of longitudinal slots (34) defining converging resilient tongues (43) .

8. A device according to any one of previous claims, **characterized in that** the sleeve (10) has grip means at its proximal end for gripping the head (6) of the syringe (2) and suitable for gripping syringes of at least two different types.

9. A device according to claim 8, **characterized in that** the grip means are arranged to grip a collar (6) forming the head of the syringe (2).

10. A device according to any one of previous claims, **characterized in that** it includes a resilient element (37) acting between the sheath (20) and the assembly constituted by the sleeve (10) and the syringe (2), and enabling said assembly to be caused to move, at the end of injection, from the first position into the second position by a rearward movement of said assembly relative to the sheath (20).

11. An assembly comprising a syringe (2) and a safety device (1) fitted to said syringe (2), the assembly being **characterized in that** said safety device (1) is in accordance with any one of claims 1 to 10.

12. An assembly according to claim 11, **characterized in that** it further includes a cap (19) protecting the needle (5) of the syringe (2).

13. An assembly according to claim 12, **characterized in that** said cap (19) is mounted in such a manner as to project beyond the sheath (20) through the distal opening (36) thereof.

14. An assembly according to claim 13, **characterized in that** the sheath (20) and the cap (19) are arranged in such a manner as to act, after the cap (19) has been removed, to prevent the cap from being reinserted through the opening (36) of the sheath (20).

## Patentansprüche

1. Sicherheitsvorrichtung (1) für eine Spritze (2) mit Nadel (5), die eine Hülse (10) zum Aufnehmen des Körpers (3) der Spritze (2) aufweist und eine Schutzhülle (20), in der die Spritze (2) gleiten kann, und Mittel, um die Spritze (2) und die Hülse in der Hülle (20) gleiten zu lassen zwischen einer ersten Position, die die Injektion ermöglicht, und einer zweiten Position, in der die Nadel (5) der Spritze (2) ins Innere der Hülle (20) zurückgezogen ist, wobei die Hülle (20) an einem distalen Teil (33), der in radialer Richtung elastisch verformbar ist, eine Öffnung (36) aufweist, um zu ermöglichen, dass die Nadel (5) der Spritze (2) aus der Hülle (20) herausragt, **dadurch gekennzeichnet, dass** die Hülle (20), an ihrem distalen Teil (33), in radialer Richtung elastisch verformbar ist, zwischen einer Ruhestellung, in der ihre Öffnung (36) einen minimalen Durchmesser aufweist, und einer verformten Stellung, die, durch die Öffnung (36) hindurch, einen Körper (3, 19) mit einem Durchmesser passieren lässt, der größer ist als der minimale Durchmesser der Öffnung (36).

2. Vorrichtung nach Anspruch 1, **dadurch gekennzeichnet, dass** der Körper (19) eine Kappe ist, dazu bestimmt, die Nadel (5) der Spritze (2) zu schützen.

3. Vorrichtung nach Anspruch 1, **dadurch gekennzeichnet, dass** der Körper (3) der Körper der Spritze (2) ist.

4. Vorrichtung nach Anspruch 1, **dadurch gekennzeichnet, dass** der Körper, wobei die Hülle durch Formung ausgebildet ist, ein beweglicher Teil einer Form ist.

5. Vorrichtung nach einem der Ansprüche 1 bis 4, **dadurch gekennzeichnet, dass** die Hülle (20), an ihrem distalen Teil (33), kegelstumpfförmig ist.

6. Vorrichtung nach einem der Ansprüche 1 bis 5, **dadurch gekennzeichnet, dass** der distale Teil (33) der Hülle (20) gespalten ist.

7. Vorrichtung nach Anspruch 6, **dadurch gekennzeichnet, dass** der distale Teil (33) der Hülle (20) mehrere Längsspalte (34) aufweist, welche konvergente elastische Zungen (43) festlegen.

8. Vorrichtung nach einem der vorstehenden Ansprüche, **dadurch gekennzeichnet, dass** die Hülse (10) an ihrem proximalen Teil Mittel zum Halten des Kopfteils (6) der Spritze (2) aufweist, die sich eignen, um Spritzen zumindest zweier verschiedener Typen zu halten.

9. Vorrichtung nach Anspruch 8, **dadurch gekennzeichnet, dass** die Haltemittel angeordnet sind, um den Halt eines das Kopfteil der Spritze (2) bildenden Kragens (6) zu gewährleisten.

10. Vorrichtung nach einem der vorstehenden Ansprüche, **dadurch gekennzeichnet, dass** sie ein elastisches Element (37) aufweist, das zwischen der Hülle (20) und der aus der Hülse (10) und der Spritze (2) bestehenden Anordnung wirkt, und das es ermöglicht, die Anordnung, am Ende der Injektion, von der ersten Position in die zweite zu überführen durch eine relative Zurückbewegung dieser Anordnung in Bezug auf die Hülle (20).

11. Anordnung, die eine Spritze (2) und eine die Spritze (2) ausstattende Sicherheitsvorrichtung (1) aufweist, **dadurch gekennzeichnet, dass** die Sicherheitsvorrichtung (1) einem der Ansprüche 1 bis 10 entspricht.

12. Anordnung nach Anspruch 11, **dadurch gekennzeichnet, dass** sie weiterhin eine Kappe (19) aufweist, die die Nadel (5) der Spritze (2) schützt.

13. Anordnung nach Anspruch 12, **dadurch gekennzeichnet, dass** die Kappe (19) so angebracht ist, dass sie aus der Hülle (20) durch die distale Öffnung (36) derselben herausragt.

14. Anordnung nach Anspruch 13, **dadurch gekennzeichnet, dass** die Hülle (20) und die Kappe (19) so angeordnet sind, dass sie, nach Herausziehen der Kappe (19), die erneute Einführung derselben durch die Öffnung (36) der Hülle (20) verhindern.
